# EUROPÄISCHE PATENTSCHRIFT

(11) **EP 1 088 208 B1**
(45) Veröffentlichungstag und Bekanntmachung des Hinweises auf die Patenterteilung: **12.05.2004**
(21) Anmeldenummer: 99929212.1
(22) Anmeldetag: 15.06.1999
(51) Int. Cl.: G01K 13/00

(54) **GERÄT ZUR DURCHFÜHRUNG VON MESSUNGEN IM OHR**
DEVICE FOR CARRYING OUT MEASUREMENTS IN THE EAR
APPAREIL POUR EFFECTUER DES MESURES DANS L'OREILLE

(30) Priorität: 19.06.1998 DE 19827343
(43) Veröffentlichungstag der Anmeldung: 04.04.2001
(73) Patentinhaber: Braun GmbH, 61476 Kronberg (DE)
(72) Erfinder: KRAUS, Bernhard, D-35619 Braunfels (DE); KAHLER, Elke, D-64347 Griesheim (DE); KLÖS, Alexander, D-65719 Hofheim (DE); MANNEBACH, Horst, D-35510 Butzbach (DE)
(86) Internationale Anmeldenummer: PCT/EP1999/004115
(87) Internationale Veröffentlichungsnummer: WO 1999/067611

(56) Entgegenhaltungen:
- EP-A- 0 588 631
- US-A- 4 567 881
- US-A- 5 325 863
- US-A- 5 419 312
- US-A- 5 626 147
- US-A- 5 658 235
- US-A- 5 673 692
- US-A- 5 790 586

## Beschreibung

Die vorliegende Erfindung betrifft ein Gerät zur Durchführung von Messungen im Ohr mit einer in den Ohrkanal einführbaren Meßspitze und einer Anzeige. Derartige Meßgeräte, beispielsweise Infrarot-Strahlungsthermometer oder Ohr-Reflektometer sind bekannt.

Aus der US 4,567,881 ist ein Gerät bekannt, das eine Kombination eines Audiometers mit einem Otoskop darstellt. Mit dem Audiometer können vier verschiedene Töne ins Ohr eines Patienten ausgesendet werden, die sich in der Lautstärke unterscheiden. Das Aussenden der vier Töne wird durch je eine Leuchtdiode angezeigt. Wenn der Patient den Finger hebt, wird davon ausgegangen, daß er den Ton gehört hat. Das Otoskop bietet gleichzeitig die Möglichkeit, ins Ohr hineinzusehen.

Aus der WO 98/01730 ist ein Infrarot-Thermometer mit einem optischen Markierungssystem bekannt, das es einem Benutzer erlaubt, bei einer Temperaturmessung einen bestimmten Abstand zwischen der Meßspitze und dem Meßobjekt einzuhalten.

Es sind aber auch Infrarot-Strahlungsthermometer zur Bestimmung der Körpertemperatur eines Menschen bekannt, deren eine Strahlungseintrittsöffnung aufweisende Meßspitze in den Gehörgang eingeführt, und die vom Trommelfell ausgestrahlte Infrarotstrahlung gemessen werden kann. Da das Trommelfell die gleiche Blutzufuhr wie das Temperaturzentrum im Gehirn besitzt, ist diese Strahlung ein genauer Indikator für die Körperkerntemperatur des Menschen.

Da üblicherweise eine Differenz zwischen der zu messenden Temperatur des Trommelfells und der Temperatur des umliegenden Gehörganggewebes besteht, ist für eine exakte Temperaturmessung eine korrekte Ausrichtung der Meßspitze auf das Trommelfell erforderlich. Dies ist jedoch nicht immer gewährleistet, da Biegungen des Gehörgangs oder häufig vorhandene natürliche Unebenheiten (Exostosen) den freien Blick auf das Trommelfell versperren können. Das Meßergebnis wird somit merklich durch die Geometrie des jeweiligen Gehörgangs beeinflußt, so daß sich mehr oder weniger starke Meßfehler ergeben können. Auch ein subjektiv korrekter Sitz der Meßspitze ist hierbei keine Garantie für eine richtige Ausrichtung auf das Trommelfell, da im ungünstigsten Fall bereits eine teilweise Abdeckung der Strahlungseintrittsöffnung durch eine Hautfalte zu einer starken Richtungsabhängigkeit der gemessenen Temperatur und damit üblicherweise zu einer für den Benutzer nicht erkennbaren Fehlmessung mit einer schlechten Reproduzierbarkeit führt. Durch versehentliches direktes Aufsetzen der Meßspitze auf das Gehörganggewebe kann es auch zu Meßfehlern kommen, die eine wesentlich zu tiefe Körpertemperatur vortäuschen.

Zur Lösung dieses Problems wird in der US-A-5,325,863 vorgeschlagen, dem Benutzer eine akustische Rückmeldung über die Qualität der Positionierung zu geben. So führt das bekannte Ohr-Fieberthermometer immer mehrere Messungen durch und besitzt einen akustischen Signalgeber, der schon beim Einführen der Meßspitze in den Gehörgang immer dann einen Ton abgibt, wenn der gemessene Temperaturwert höher ist als ein vorheriger Meßwert. Die Auswertung der temperaturmäßig erfaßten Bereiche ist hierbei jedoch zufallsabhängig und es gibt keine Kontrollmöglichkeit, ob das Trommelfell als der insgesamt wärmste Bereich auch in die Bewertung mit einbezogen und die Körperkemtempertur auch richtig erfaßt wurde. Zudem lassen sich auch Fehlmessungen, die durch direktes Aufsetzen der Meßspitze auf Gewebe bedingt sind, nicht erkennen und daher auch nicht während der Durchführung einer Messung korrigieren.

Aus der US 5,626,147 ist ein Ohrthermometer mit einer Einrichtung bekannt, mit der Temperatur-Meßfehler kompensiert werden können, die durch ein falsches Orientieren der Meßspitze im Ohrkanal hervorgerufen werden. Diese Einrichtung enthält mindestens zwei Infrarot-Sensoren und eine Auswerteeinheit, die aus den Ausgangssignalen der beiden Infrarot-Sensoren und Temperatur-Korrekturwerten einen Temperatur-Meßwert bestimmt. Die Temperatur-Korrekturwerte sind entweder in der Auswerteeinheit gespeichert oder werden von der Auswerteeinheit nach einem nicht näher beschriebenen Algorithmus berechnet, wobei der Ausgangspunkt für diese Berechnung die Differenz der Ausgangssignale der beiden Infrarot-Sensoren ist. Bei diesem Ohrthermometer wird die Ausrichtung der Meßspitze im Ohrkanal weder bestimmt noch angezeigt.

Genau dieselben Probleme hinsichtlich der korrekten Ausrichtung der Meßspitze im Ohrkanal treten bei der Messung der Impedanz des Trommelfells mit Hilfe eines Ohr-Reflektometers auf, mit dem Flüssigkeitsansammlungen im Mittelohr festgestellt werden können. Dazu werden Schallwellen mit verschiedenen Frequenzen ausgesendet, und die am Trommelfell reflektierten Schallwellen registriert und ausgewertet.

Die Aufgabe der vorliegenden Erfindung besteht daher in der Schaffung eines Geräts zur Durchführung von Messungen im Ohr, bei dem fehlerhafte Meßwerte aufgrund ungenügender Ausrichtung der Meßspitze aufs Trommelfell vermieden werden können.

Diese Aufgabe wird erfindungsgemäß durch ein Gerät gelöst, das eine Einrichtung zur Bestimmung der Ausrichtung der Meßspitze im Ohrkanal aufweist.

Diese Einrichtung umfaßt eine elektromagnetische Strahlung emittierende Strahlungsquelle zum Anstrahlen des von der Meßspitze des Geräts anvisierten Meßflecks, einen im entsprechenden Wellenlängenbereich ansprechenden Detektor zur Erfassung der von dem Meßfleck gestreuten Strahlung, und eine dem Detektor nachgeschaltete Auswerteeinrichtung zur Bestimmung der Ausrichtung der Meßspitze im Ohrkanal. Sie kann ferner auch noch eine Anzeigeeinrichtung zur Darstellung der Ausrichtung enthalten.

Bei einer bevorzugten Ausführung eines erfindungsgemäßen Geräts ist die Strahlungsquelle eine Lichtquelle, die vorzugsweise im sichtbaren Bereich oder im nahen Infrarot emittiert, und der Detektor besteht dann aus einem Photodetektor.

Um eine Beeinflussung der Messung durch die in den Gehörgang eingestrahlte Strahlung auszuschließen, wird die mittlere Intensität der Strahlungsquelle entsprechend klein gewählt, sodaß es dadurch insbesondere nicht zu einer Erwärmung des Gehörgangs kommt. Dies läßt sich vorzugsweise durch Verwendung einer gepulsten Strahlungsquelle und einer mit dieser synchronisierten Detektorschaltung erreichen. Eine gepulste Strahlungsquelle hat eine geringere Leistungsaufnahme als eine nichtgepulste, was insbesondere bei batteriebetriebenen Geräten von Vorteil ist. Außerdem läßt sich bei einer gepulsten Strahlungsquelle auch der Untergrund der Umgebungsstrahlung bestimmen.

Ein erfindungsgemäßes Gerät weist vorteilhafterweise einen ersten Strahlungsleiter auf, bei obiger Ausführung einen Lichtleiter, der die von der Strahlungsquelle emittierte Strahlung zum Meßfleck hin leitet, d.h. die Strahlung tritt am dem Meßfleck zugewandten Ende des Strahlungsleiters aus und strahlt in den Gehörgang. Die Strahlung kann gebündelt sein oder einen sich aufweitenden Strahlungskegel bilden. Trifft die Strahlung auf Gewebe auf, wird sie diffus reflektiert, wodurch ein Teil entweder über denselben oder einen zweiten Strahlungsleiter zum Detektor gelangt. Die Intensität der vom Detektor erfaßten Strahlung ist vom mittleren Abstand des reflektierenden Gewebes von der Meßspitze abhängig. Bei einer Fehlausrichtung der Meßspitze wird daher ein relativ großer Anteil der Strahlung nach Streuung an den Wänden des Gehörgangs wieder in die Meßspitze eingekoppelt und im Detektor detektiert, während bei einer korrekten Ausrichtung der Meßspitze auf den hinteren Gehörgang und das Trommelfell nur eine minimale Strahlungsmenge detektiert wird. Dadurch lassen sich auch gravierende Fehlmessungen erkennen, wie sie beispielsweise bei direktem Aufsetzen der Meßspitze auf Gewebe auftreten können.

Bei der oben beschriebenen Verwendung zweier Strahlungsleiter ist eine Falschanzeige durch direktes Überkoppeln der emittierten Strahlung zum Detektor ausgeschlossen.

Bei einer anderen Ausführung eines erfindungsgemäßen Meßgeräts fehlt der erste Strahlungsleiter. Die Strahlungsquelle ist dann ganz vome in der Meßspitze angeordnet.

Zur Anleitung des Benutzers hinsichtlich einer korrekten Ausrichtung der Meßspitze kann die Ausrichtung der Meßspitze in optischer und/oder akustischer Form angezeigt werden. Dazu mißt die erfindungsgemäße Einrichtung fortlaufend, d.h. vorzugsweise schon beim Einführen der Meßspitze in den Ohrkanal, die gestreute Strahlungsmenge. Vorzugsweise umfaßt die Anzeigeeinrichtung eine Einrichtung zur Erzeugung eines akustischen Signals, dessen Lautstärke und/oder Frequenz durch das Ausgangssignal des Detektors gesteuert wird. Der Benutzer eines erfindungsgemäßen Geräts erhält bereits kurz nach dem Einführen der Meßspitze in den Gehörgang eine unmittelbare Rückmeldung über die optimale Ausrichtung der Meßspitze auf das Trommelfell oder zumindest in den Bereich des hinteren Gehörgangs. Diese Anzeige führt dazu, daß der Benutzer bei jeder Messung die Meßspitze nahezu gleich ausrichtet, sodaß sich eine gute Reproduzierbarkeit der Meßergebnisse ergibt.

Die Anzeigeeinrichtung enthält beispielsweise einen durch das Ausgangssignal des Detektors angesteuerten spannungsgesteuerten Oszillator, an den ein Lautsprecher angeschlossen ist. Eine Änderung der detektierten Strahlungsleistung bewirkt dann eine Erhöhung oder Erniedrigung der Schwingungsfrequenz und/oder -amplitude, so daß der Benutzer bei der Ausrichtung des Thermometers im Ohrkanal einen sich entsprechend verändernden Ton hört. Die korrekte Ausrichtung der Meßspitze ist dann erreicht, wenn der Ton mit der niedrigsten (bzw. höchsten) Frequenz und/oder Amplitude zu hören ist.

Um eine Messung durch Dritte zu erleichtern, kann die Anzeigeeinrichtung in einer anderen Ausführungsform jedoch auch eine optische Anzeige, insbesondere eine LED-Zeile oder ein Zeigerinstrument umfassen, durch die dem Benutzer die korrekte Ausrichtung der Meßspitze zum hinteren Gehörgang und zum Trommelfell angezeigt wird. Diese Art der Darstellung ermöglicht eine Messung auch an schlafenden Personen, ohne daß es hierbei zu einer Störung durch ein akustisches Signal kommt.

Bei einer bevorzugten Ausführungsform weist die Auswerteeinrichtung eines erfindungsgemäßen Geräts zusätzlich noch eine Schwellwerteinrichtung auf, die erst bei Unterschreiten eines bestimmten Schwellwerts für die im Detektor detektierte Strahlungsleistung die Anzeigeeinrichtung für die Meßwerte und/oder die Anzeigeeinrichtung zur Darstellung der Ausrichtung aktiviert. Somit kann die Anzeige eines Meßwerts solange unterdrückt werden, bis die Auswerteeinrichtung eine zufriedenstellende Ausrichtung der Meßspitze erkannt hat. Femer kann bei Erreichen des Schwellwerts dem Benutzer das Ende der Messung angezeigt werden. Der Schwellwert ist so festgelegt, daß sich auch bei unterschiedlich geformten Ohrkanälen die erforderliche Meßgenauigkeit ergibt.

Bei einem besonders vorteilhaft ausgestalteten Meßgerät wird der Schwellwert bereits beim Einführen der Meßspitze in den Ohrkanal an das individuelle Streuvermögen des jeweiligen Ohrkanals angepaßt. Dies gelingt besonders gut, wenn der Benutzer die Meßspitze zunächst im Ohrkanal hin- und herbewegt und dadurch für unterschiedliche Ausrichtungen der Meßspitze sorgt. Die Schwellwerteinrichtung erkennt das Hin- und Herbewegen an entsprechenden Schwankungen des Ausgangssignals des Detektors und ändert den Schwellwert; wenn trotz dieses Bewegens innerhalb einer festgelegten Meßzeit der Schwellwert nicht erreicht wird.

Eine Fehlbedienung durch direktes Aufsetzen der Meßspitze auf Gewebe wird angenommen, wenn das Ausgangssignal des Detektors einen entsprechend festgelegten zweiten Schwellwert erreicht.

Zwei bevorzugte Ausführungsbeispiele für ein erfindungsgemäßes Meßgerät sind ein Infrarot-Strahlungsthermometer mit einem Infrarot-Sensor, insbesondere zur Messung der Temperatur des Trommelfells, und ein Ohr-Reflektometer mit einer Schallquelle und einem Mikrophon, insbesondere zur Messung der Impedanz des Trommelfells.

Bekannte Infrarot-Strahlungsthermometer weisen üblicherweise ein Gehäuse mit einer Strahlungseintrittsöffnung für die zu messende Infrarotstrahlung sowie einen Wellenleiter auf, der sich von der Strahlungseintrittsöffnung bis zum Infrarot-Sensor erstreckt. Bei einem erfindungsgemäßen Infrarot-Strahlungsthermometer dient der Wellenleiter zur Weiterleitung sowohl der vom Trommelfell ausgehenden Infrarotstrahlung zum Infrarot-Sensor als auch der im Gehörgang reflektierten Strahlung der Strahlungsquelle zum Detektor. Daher sind der Infrarot-Sensor und der Detektor vorzugsweise am Ende des Wellenleiters (der somit auch die Funktion des obengenannten zweiten Strahlungsleiters übemimmt) angeordnet.

In einer anderen Ausführung sind für die Infrarotstrahlung und die reflektierte Strahlung zwei separate Strahlungsleiter vorgesehen. Alternativ kann die zu messende Infrarotstrahlung, die ausgestrahlte und die reflektierte Strahlung in einem einzigen Strahlungsleiter geführt werden. Statt des(der) Strahlungsleiter(s) kann auch ein optisches System mit Linsen und/oder Spiegeln vorgesehen sein.

Der Detektor ist vorzugsweise seitlich im Endbereich des Wellenleiters angeordnet, um seinen Einfluß auf den Blickwinkel und die Empfindlichkeit des Infrarot-Sensors möglichst gering zu halten. Er kann aber auch im Gehäuse des Infrarot-Sensors integriert sein, wobei dann das Fenstermaterial des Sensorgehäuses so gewählt ist, daß das Fenster sowohl für die Infrarotstrahlung als auch im von der Strahlungsquelle verwendeten Wellenlängenbereich durchlässig ist. In einer besonderen Ausgestaltung ist der Detektor mit dem Infrarot-Sensor in einem Mikrosystem auf einem Chip integriert. Auch eine Integration von Infrarot-Sensor, Detektor und Strahlungsquelle auf einem Chip oder in einem Gehäuse ist möglich.

Bei einem erfindungsgemäßen Ohr-Reflektometer können der Detektor und das Mikrophon ein gemeinsames Gehäuse aufweisen. In einer besonderen Ausgestaltung ist der Detektor mit dem Mikrophon in einem Mikrosystem auf einem Chip integriert. Auch eine Integration von Mikrophon, Detektor und Strahlungsquelle auf einem Chip oder in einem Gehäuse ist möglich.

Die Erfindung wird nachfolgend anhand bevorzugter Ausführungsbeispiele in Verbindung mit den zugehörigen Figuren erläutert. Weitere Ausführungen sind in der Beschreibung enthalten. Es zeigen in schematischer Darstellung:
- Fig. 1: eine im Gehörgang eines Benutzers fehlerhaft ausgerichtete Meßspitze eines erfindungsgemäßen Infrarot-Strahlungsthermometers;
- Fig. 2: die korrekt ausgerichtete Meßspitze gemäß Fig. 1;
- Fig. 3: die Komponenten der erfindungsgemäßen Einrichtung zur Bestimmung der Ausrichtung der Meßspitze beim einem Infrarot-Strahlungsthermometer;
- Fig. 4: eine im Gehörgang eines Benutzers fehlerhaft ausgerichtete Meßspitze eines erfindungsgemäßen Ohr-Reflektometers;
- Fig. 5: die korrekt ausgerichtete Meßspitze gemäß Fig. 4;
- Fig. 6: die Komponenten der erfindungsgemäßen Einrichtung zur Bestimmung der Ausrichtung der Meßspitze und die Komponenten für die Ohr-Reflektometrie.

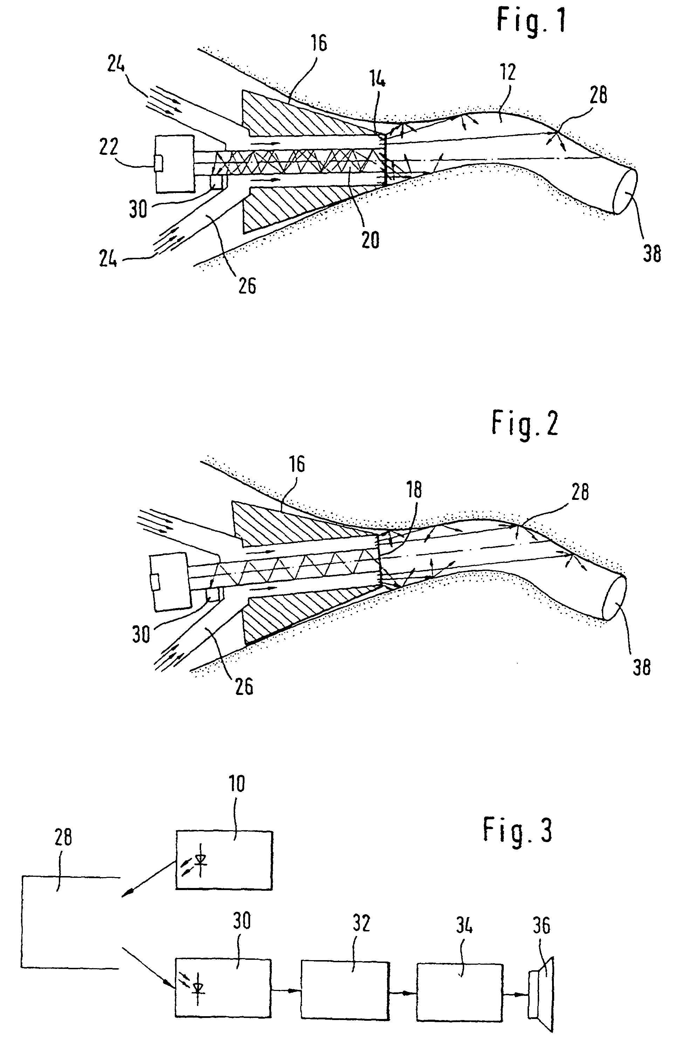

Die Figuren 1 und 2 zeigen schematisch eine Meßspitze eines erfindungsgemäßen Infrarot-Strahlungsthermometers, die zur Durchführung einer Fiebermessung in den Ohrkanal 12 eines Benutzers eingeführt wurde. Die Meßspitze umfaßt bekanntermaßen ein sich zu einer Strahlungseintrittsöffnung 14 für die zu messende Infrarotstrahlung hin konisch verjüngendes Gehäuse 16. Die Strahlungseintrittsöffnung 14 ist durch ein Strahlungseintrittsfenster 18 abgeschlossen, um das Gehäuseinnere gegen Verschmutzung und Beschädigung zu schützen.

Der vordere Teil der Meßspitze kann mit einer (nicht dargestellten) austauschbaren Schutzkappe abgedeckt sein, um insbesondere eine Übertragung von Krankheiten von Patient zu Patient zu verhindern. Sie besteht beispielsweise aus einer dünnen Polyethylenfolie.

Die in die Strahlungseintrittsöffnung 14 einfallende Infrarotstrahlung wird in an sich bekannter Weise durch einen Wellenleiter 20 zu einem Infrarot-Sensor 22 geleitet. Der Infrarot-Sensor 22 ist mit einer (nicht dargestellten) Auswerteelektronik verbunden, von der die erfaßte Infrarotstrahlung in eine Temperaturangabe umgewandelt und mittels einer (ebenfalls nicht dargestellten) Anzeigeeinrichtung für die Meßwerte angezeigt wird.

Darüber hinaus enthält die Meßspitze erfindungsgemäß eine Einrichtung zur Bestimmung der Ausrichtung der Meßspitze im Ohrkanal, die eine beispielsweise im sichtbaren Bereich oder im nahen Infrarot emittierende Lichtquelle 10 (Fig. 3) aufweist, deren Licht 24 durch einen den Wellenleiter 20 konzentrisch umgebenden ersten Strahlungsleiter 26 zur Strahlungseintrittsöffnung 14 geleitet wird. Von hier aus strahlt es den Ohrkanal 12 an, und wird von dessen Wänden 28 diffus reflektiert, was in den Figuren durch Pfeile dargestellt ist. Ein Teil dieser gestreuten Strahlung wird über die Strahlungseintrittsöffnung 14 in die Meßspitze zurückgekoppelt und über den Wellenleiter 20 zu einem Photodetektor 30 geleitet, der seitlich im hinteren Teil des Wellenleiters 20 angeordnet ist und ein entsprechendes elektrisches Signal abgibt. Dieses wird einer (in den Figuren nicht dargestellten) Auswerte- und bei einer alternativen Ausführung auch einer Anzeigeeinrichtung zugeführt, die dem Benutzer die Ausrichtung der Meßspitze im Ohrkanal auf die weiter unten beschriebene Art in akustischer und/oder optischer Form anzeigt.
Bei einer anderen Ausführung ist der erste Strahlungsleiter 26 zumindest teilweise im Wellenleiter 20 angeordnet.

Um eine merkliche Erwärmung des Ohrkanals 12 und eine damit verbundene Beeinflussung der Temperaturmessung auszuschließen, wird die mittlere Intensität des in den Ohrkanal 12 eingestrahlten Lichts 24 entsprechend gering gewählt. Dazu wird in an sich bekannter Weise die Lichtquelle 10 im Pulsbetrieb betrieben, und der Photodetektor 30 mit dieser synchronisiert.

Bei einer anderen Ausführung ist der Photodetektor 30 im Gehäuse des Infrarot-Sensors 22 angeordnet, wobei das Fenstermaterial des Sensorgehäuses so gewählt ist, daß es nicht nur für die zu messende Infrarotstrahlung sondern auch für das von der Lichtquelle 10 emittierte Licht 24 durchlässig ist. Bei einer weiteren Ausführung sind der Infrarot-Sensor 22 und der Photodetektor 30 in einem Mikrosystem auf einem Chip integriert. Bei anderen Varianten ist die Strahlungsquelle 10 mit dem Infrarot-Sensor und/oder Detektor auf einem Chip integriert bzw. in einem Gehäuse angeordnet.

In einer in den Figuren nicht dargestellten anderen Ausführungsform wird das in die Strahlungseintrittsöffnung 14 der Meßspitze eingekoppelte Licht nicht durch den Wellenleiter 20 sondern durch einen zusätzlichen Strahlungsleiter zum Photodetektor 30 geleitet, um eine mögliche Beeinflussung des Blickfelds des Infrarot-Sensors 22 durch den Photodetektor 30 zu vermeiden.

Da in der Meßspitze das von der Lichtquelle 10 emittierte Licht 24 im ersten Strahlungsleiter 26 getrennt von der vom Ohrkanal ausgehenden Strahlung im Wellenleiter 20 geführt ist, ist gewährleistet, daß von der Lichtquelle emittiertes Licht, das am Strahlungseintrittsfenster 18 bzw. an der (nicht dargestellten) Schutzkappe reflektiert wird, den Photodetektor 30 nicht erreichen kann. Auch bei einer falschen Handhabung des erfindungsgemäßen Infrarot-Fieberthermometers durch direktes Aufsetzen der Meßspitze auf Gewebe wird daher aufgrund dessen Transparenz für das von der Lichtquelle emittierte Licht nur am Gewebe reflektiertes Licht in ausreichender Intensität zum Photodetektor 30 zurückgekoppelt, so daß eine Fehlausrichtung der Meßspitze erkannt und vermieden werden kann.

Da das in den Ohrkanal 12 eingestrahlte Licht dort diffus reflektiert wird, ist die Intensität der vom Photodetektor 30 detektierten Strahlung vom mittleren Abstand der Meßspitze vom Ohrkanal 12 abhängig. Bei einer Fehlausrichtung der Meßspitze, wie sie beispielsweise in Fig. 1 dargestellt ist, wird ein relativ großer Anteil des in den Ohrkanal eingestrahlten Lichts nach Streuung an den Wänden 28 des Ohrkanals 12 wieder in die Meßspitze zurückgekoppelt und im Photodetektor 30 detektiert. Bei der in Fig. 2 dargestellten korrekten Ausrichtung der Meßspitze im Ohrkanai 12 wird hingegen nur ein kleiner Teil des an den Wänden 28 des Ohrkanals 12 gestreuten Lichts im Photodetektor 30 detektiert. Die Meßspitze des Infrarot-Thermometers ist in diesem Fall auf den hinteren Ohrkanal 12 und das Trommelfell 38 gerichtet, sodaß der Infrarot-Sensor 22 überwiegend die vom Trommelfell ausgehende Strahlung empfängt, und die korrekte Körperkerntemperatur des Benutzers gemessen wird.

Wie in Fig. 3 schematisch dargestellt ist, emittiert die Strahlungsquelle 10 Strahlung, die an den Wänden 28 des Ohrkanals gestreut wird und zumindest teilweise zum Detektor 30 gelangt, dessen Ausgangssignal in einem Verstärker 32 verstärkt und einem spannungsgesteuerten Oszillator (VCO) 34 zugeführt wird. Das Ausgangsignal des Oszillators 34 wird dem Benutzer über einen Lautsprecher 36 hörbar gemacht. Da eine höhere detektierte Strahlungsleistung ein größeres Signal des Photodetektors 30 zur Folge hat, ergibt sich dadurch auch eine entsprechend andere Schwingungsfrequenz des Oszillators. Der Benutzer hört daher bei einem Hin- und Herbewegen der Meßspitze im Ohr aufgrund der dadurch bedingten Veränderung der gemessenen Strahlungsleistung einen an- und abschwellenden Ton. Die in Fig. 2 dargestellte korrekte Ausrichtung der Meßspitze ist dann erreicht, wenn der Ton mit der niedrigsten bzw. höchsten Frequenz zu hören ist.

Der Benutzer bekommt somit bei jeder Veränderung der Ausrichtung der Meßspitze im Ohr eine direkte akustische Rückmeldung, die ihm eine korrekte Ausrichtung der Meßspitze auf den hinteren Bereich des Gehörgangs 12 ermöglicht, dessen Temperatur der Temperatur des Trommelfells 38 entspricht oder nur geringfügig von dieser abweicht. Die erfindungsgemäße Einrichtung hilft somit dem Benutzer die korrekte Ausrichtung der Meßspitze im Ohrkanal zu finden und gewährleistet unabhängig von der jeweiligen Größe und Form des Ohrkanals eine optimale Ausrichtung der Meßspitze bei jeder Messung, sodaß sich eine gute Wiederholgenauigkeit der Meßergebnisse ergibt.

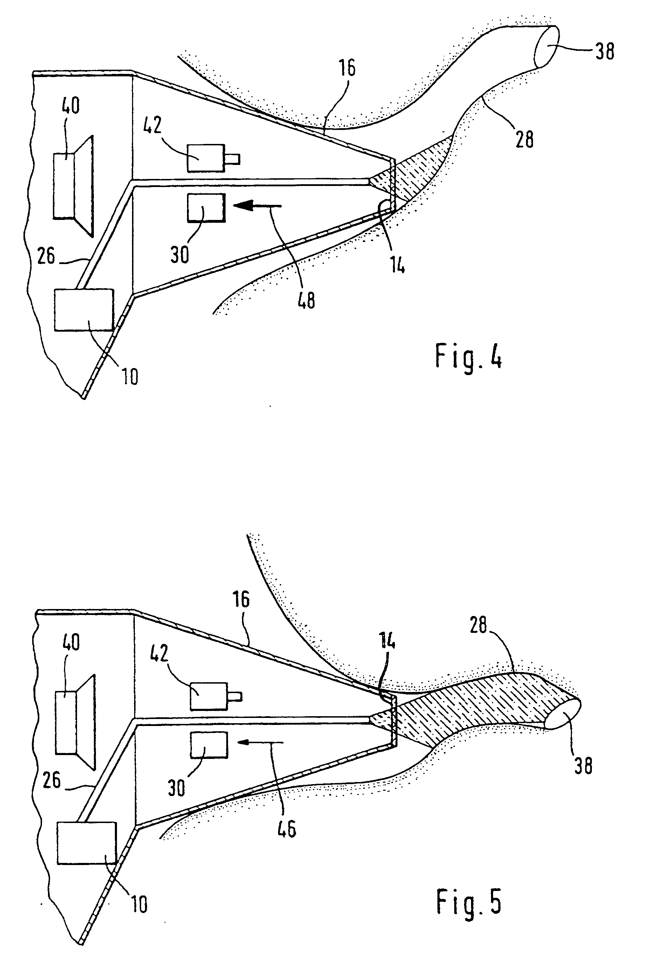

In den Figuren 4 und 5 ist schematisch eine Meßspitze eines mit einer erfindungsgemäßen Einrichtung zur Bestimmung der Ausrichtung der Meßspitze ausgerüsteten Ohr-Reflektometers dargestellt, die in einen Ohrkanal eingeführt ist. Sie ist ähnlich wie die in den Figuren 1 und 2 dargestellte Meßspitze aufgebaut, enthält jedoch statt des Infrarot-Sensors 22 in an sich bekannter Weise eine Schallquelle 40 und ein Mikrophon 42, die beide in Richtung einer Strahlungseintrittsöffnung 14 ausgerichtet sind. Ferner enthält sie keinen Wellenleiter 20, sondern nur den ersten Strahlungsleiter 26, durch den von der Strahlungsquelle 10 emittierte Strahlung zur Strahlungseintrittsöffnung 14 geleitet wird. Eine andere erfindungsgemäße Ausführung weist jedoch auch einen zweiten Strahlungsleiter auf, durch den vom Ohrkanal reflektierte Strahlung zum Detektor 30 geleitet wird. Alternativ kann die ausgestrahlte und reflektierte Strahlung in einem einzigen Strahlungsleiter geführt werden. Ansonsten sind in den Figuren 4, 5 und 6 für gleiche Bauteile dieselben Bezugszeichen verwendet wie in den Figuren 1, 2 und 3, sodaß auf eine sich wiederholende Beschreibung verzichtet werden kann.

In Fig. 4 ist schematisch eine nicht korrekt im Ohrkanal ausgerichtete Meßspitze dargestellt, bei der am Detektor 30 eine entsprechend hohe Intensität reflektierter Strahlung festgestellt wird, die durch einen dicken Pfeil 48 symbolisiert ist, wogegen Fig. 5 eine korrekt ausgerichtete Meßspitze zeigt, deren Detektor 30 nur eine vergleichsweise geringe Strahlungsteistung detektiert, die durch einen dünnen Pfeil 46 symbolisiert ist.

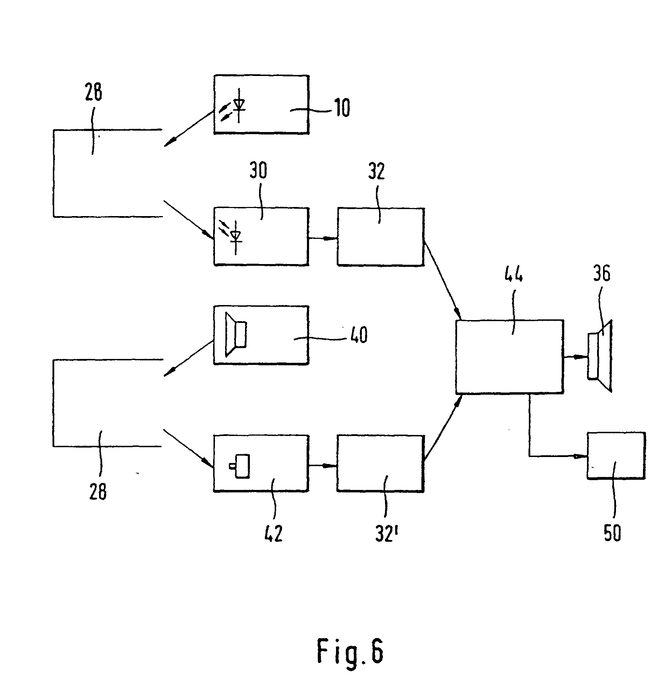

Wie in Fig. 6 schematisch dargestellt ist, werden bei einem erfindungsgemäßen Ohr-Reflektometer durch einen Prozessor 44 sowohl das Ausgangssignal des dem Detektor 30 nachgeschalteten Verstärkers 32 als auch die in einem Verstärker 32' verstärkten Ausgangssignale des Mikrophons 42 verarbeitet. Das Ergebnis der Reflektometrie wird vorzugsweise auf einem Display 50 dargestellt, das an den Prozessor 44 angeschlossen ist.

## Patentansprüche

1. Meßgerät zur Durchführung von Messungen im Ohr mit einer in den Ohrkanal einführbaren Meßspitze, und einer Anzeigeeinrichtung für Meßwerte,
**dadurch gekennzeichnet,**
**daß** das Gerät ferner eine Einrichtung aufweist, die die Ausrichtung der Meßspitze im Ohrkanal bestimmen kann, wobei die Einrichtung zur Bestimmung der Ausrichtung eine Strahlungsquelle (10) zum Anstrahlen eines Meßflecks mit elektromagnetischer Strahlung, einen Detektor (30) zur Erfassung der vom Meßfleck gestreuten Strahlung und eine dem Detektor (30) nachgeschaltete Auswerteeinrichtung zur Bestimmung der Ausrichtung der Meßspitze im Ohrkanal aufweist.

2. Gerät nach Anspruch 1,
**dadurch gekennzeichnet,**
**daß** es ferner eine Anzeigeeinrichtung zur Darstellung der Ausrichtung der Meßspitze im Ohrkanal aufweist.

3. Gerät nach Anspruch 1 oder 2,
**dadurch gekennzeichnet,**
**daß** die Strahlungsquelle (10) eine Lichtquelle, und der Detektor ein Photodetektor (30) ist.

4. Gerät nach einem der Ansprüche 1 bis 3,
**dadurch gekennzeichnet,**
**daß** die Strahlungsquelle (10) gepulste Strahlung abgibt, und der Detektor (30) mit der Strahlungsquelle (10) synchronisiert ist.

5. Gerät nach einem der Ansprüche 1 bis 4,
**dadurch gekennzeichnet,**
**daß** die Strahlungsquelle (10) und der Detektor (30) in einem gemeinsamen Gehäuse angeordnet sind.

6. Gerät nach einem der Ansprüche 1 bis 5,
**dadurch gekennzeichnet,**
**daß** die Strahlungsquelle (10) auf demselben Chip integriert ist wie der Detektor (30).

7. Gerät nach einem der Ansprüche 1 bis 6,
**dadurch gekennzeichnet,**
**daß** es einen Strahlungs-, insbesondere Lichtleiter (26) für die von der Strahlungsquelle (10) ausgehende Strahlung enthält.

8. Gerät nach einem der Ansprüche 1 bis 7,
**dadurch gekennzeichnet,**
**daß** es einen Strahlungs-, insbesondere Lichtleiter (20) für die vom Meßfleck ausgehende Strahlung enthält, an dessen Ende der Detektor (30) angeordnet ist.

9. Gerät nach einem der Ansprüche 1 bis 8,
**dadurch gekennzeichnet,**
**daß** die Auswerteeinrichtung eine Schwellwerteinrichtung umfaßt, die erst dann eine oder beide Anzeigeeinrichtungen aktiviert, wenn das Ausgangssignal des Detektors (30) einen bestimmten Schwellwert über- oder unterschreitet.

10. Gerät nach Anspruch 9,
**dadurch gekennzeichnet,**
**daß** der Schwellwert von der Schwellwerteinrichtung veränderbar ist.

11. Gerät nach einem der Ansprüche 1 bis 10,
**dadurch gekennzeichnet,**
**daß** die Anzeigeeinrichtung zur Darstellung der Ausrichtung eine Einrichtung zur Erzeugung eines akustischen Signals umfaßt, dessen Lautstärke und/oder Frequenz durch das Ausgangssignal des Detektors (30) steuerbar ist.

12. Gerät nach einem der Ansprüche 1 bis 11,
**dadurch gekennzeichnet,**
**daß** die Anzeigeeinrichtung eine optische Anzeige, insbesondere eine LED-Zeile oder ein Zeigerinstrument umfaßt.

13. Gerät nach einem der vorhergehenden Ansprüche,
**dadurch gekennzeichnet,**
**daß** es ein Infrarot-Strahlungsthermometer mit einem Infrarot-Sensor (22) ist, insbesondere zur Messung der Temperatur des Trommelfells, oder ein Ohr-Reflektometer mit einer Schallquelle (40) und einem Mikrophon (42), insbesondere zur Messung der Impedanz des Trommelfells.

14. Infrarot-Strahlungsthermometer nach Anspruch 13 und 8,
**dadurch gekennzeichnet,**
**daß** der Infrarot-Sensor (22) am Ende des Strahlungs/Lichtleiters (20) angeordnet ist.

15. Infrarot-Strahlungsthermometer nach Anspruch 14,
**dadurch gekennzeichnet,**
**daß** der Detektor (30) und der Infrarot-Sensor (22) ein gemeinsames Gehäuse aufweisen.

16. Infrarot-Strahlungsthermometer nach Anspruch 14 oder 15,
**dadurch gekennzeichnet,**
**daß** der Detektor (30) und der Infrarot-Sensor (22) auf demselben Chip integriert sind.

17. Ohr-Reflektometer nach Anspruch 13 und 1,
**dadurch gekennzeichnet,**
**daß** der Detektor (30) und das Mikrophon (42) ein gemeinsames Gehäuse aufweisen.

18. Ohr-Reflektometer nach Anspruch 13 und 1 oder nach Anspruch 17,
**dadurch gekennzeichnet,**
**daß** der Detektor (30) und das Mikrophon (42) auf demselben Chip integriert sind.

## Claims

1. A measuring apparatus for taking measurements in the ear, having a probe head insertable into the ear canal and an indicating unit for measured values,
**characterized in that** said apparatus further includes a device capable of determining the alignment of the probe head in the ear canal, said device for determining the alignment comprising a source of radiation (10) for radiating electromagnetic radiation onto a measurement spot, a detector (30) for sensing the radiation scattered by the measurement spot, and an evaluation unit arranged downstream of the detector (30) for determining the alignment of the probe head in the ear canal.

2. The apparatus as claimed in claim 1,
**characterized in that** it further includes an indicating unit to represent the alignment of the probe head in the ear canal.

3. The apparatus as claimed in claim 1 or 2,
**characterized in that** the source of radiation (10) is a light source, and the detector is a photodetector (30).

4. The apparatus as claimed in any one of the claims 1 to 3,
**characterized in that** the radiation source (10) emits pulsed radiation, and the detector (30) is synchronized with the radiation source (10).

5. The apparatus as claimed in any one of the claims 1 to 4,
**characterized in that** the radiation source (10) and the detector (30) are arranged in a common housing.

6. The apparatus as claimed in any one of the claims 1 to 5,
**characterized in that** the radiation source (10) is integrated on the same chip as the detector (30).

7. The apparatus as claimed in any one of the claims 1 to 6,
**characterized in that** it includes a radiation guide, in particular a light guide (26), for the radiation emitted from the radiation source (10).

8. The apparatus as claimed in any one of the claims 1 to 7,
**characterized in that** it includes a radiation guide, in particular a light guide (20), for the radiation emitted from the measurement spot at the end of which is arranged the detector (30).

9. The apparatus as claimed in any one of the claims 1 to 8,
**characterized in that** the evaluation unit includes a threshold value device which activates one or both indicating units not until the output signal of the detector (30) has exceeded or fallen below a predetermined threshold value.

10. The apparatus as claimed in claim 9,
**characterized in that** the threshold value is adapted to be varied by the threshold value device.

11. The apparatus as claimed in any one of the claims 1 to 10,
**characterized in that** the indicating unit for representing the alignment comprises a device for producing an audible signal whose volume and/or frequency is/are controlled by the output signal of the detector (30).

12. The apparatus as claimed in any one of the claims 1 to 11,
**characterized in that** the indicating unit comprises a visual display, in particular an LED line or a pointer instrument.

13. The apparatus as claimed in any one of the preceding claims,
**characterized in that** it is an infrared radiation thermometer having an infrared sensor (22), particularly for measuring the temperature of the tympanic membrane, or an ear reflectometer having a sound source (40) and a microphone (42), in particular for measuring the impedance of the tympanic membrane.

14. The infrared radiation thermometer as claimed in daim 13 and 8,
**characterized in that** the infrared sensor (22) is disposed at the end of the radiation/light guide (20).

15. The infrared radiation thermometer as claimed in claim 14,
**characterized in that** the detector (30) and the infrared sensor (22) have a common housing.

16. The infrared radiation thermometer as claimed in claim 14 or 15,
**characterized in that** the detector (30) and the infrared sensor (22) are integrated on the same chip.

17. The ear reflectometer as claimed in claim 13 and 1,
**characterized in that** the detector (30) and the microphone (42) have a common housing.

18. The ear reflectometer as claimed in claim 13 and 1 or claim 17,
**characterized in that** the detector (30) and the microphone (42) are integrated on the same chip.

## Revendications

1. Appareil de mesure pour effectuer des mesures dans l'oreille à l'aide d'une sonde de mesure pouvant être introduite dans le canal auriculaire et d'un dispositif d'affichage pour les valeurs de mesure,
**caractérisé en ce que**
l'appareil présente en outre un dispositif qui peut définir l'orientation de la sonde de mesure dans le canal auriculaire, le dispositif de définition de l'orientation présentant une source de rayonnement (10) pour irradier un point de mesure avec un rayonnement électromagnétique, un détecteur (30) pour enregistrer le rayonnement diffusé par le point de mesure et un dispositif d'exploitation installé en aval du détecteur (30) pour définir l'orientation de la sonde de mesure dans le canal auriculaire.

2. Appareil selon la revendication 1, **caractérisé en ce qu'**il présente de plus un dispositif d'affichage pour représenter l'orientation de la sonde de mesure dans le canal auriculaire.

3. Appareil selon la revendication 1 ou 2, **caractérisé en ce que** la source de rayonnement (10) est une source lumineuse et le détecteur un photodétecteur (30).

4. Appareil selon l'une quelconque des revendications 1 à 3, **caractérisé en ce que** la source de rayonnement (10) émet un rayonnement en rafale et que le détecteur (30) est synchronisé à la source de rayonnement (10).

5. Appareil selon l'une quelconque des revendications 1 à 4, **caractérisé en ce que** la source de rayonnement (10) et le détecteur (30) sont disposés dans un boîtier commun.

6. Appareil selon l'une quelconque des revendications 1 à 5, **caractérisé en ce que** la source de rayonnement (10) est intégrée sur la même puce que le détecteur (30).

7. Appareil selon l'une quelconque des revendications 1 à 6, **caractérisé en ce qu'**il comporte un conducteur de rayonnement, notamment de lumière (26), pour le rayonnement émanant de la source de rayonnement (10).

8. Appareil selon l'une quelconque des revendications 1 à 7, **caractérisé en ce qu'**il comporte un conducteur de rayonnement, notamment de lumière (26), pour le rayonnement émanant du point de mesure et à l'extrémité duquel est disposé le détecteur (30).

9. Appareil selon l'une quelconque des revendications 1 à 8, **caractérisé en ce que** le dispositif d'exploitation comprend un système de valeur seuil qui n'active un ou deux dispositifs d'affichage que si le signal de sortie du détecteur (30) dépasse ou sous-dépasse une certaine valeur seuil.

10. Appareil selon la revendication 9, **caractérisé en ce que** la valeur seuil peut être modifiée par le système de valeur seuil.

11. Appareil selon l'une quelconque des revendications 1 à 10, **caractérisé en ce que** le dispositif d'affichage servant à représenter l'orientation comprend un dispositif de génération d'un signal acoustique dont l'intensité sonore et/ou la fréquence peut être commandée par le signal de sortie du détecteur (30).

12. Appareil selon l'une quelconque des revendications 1 à 11, **caractérisé en ce que** le dispositif d'affichage comprend un afficheur optique, notamment une ligne de DEL ou un instrument indicateur.

13. Appareil selon l'une quelconque des revendications précédentes, **caractérisé en ce qu'**il est un thermomètre à rayonnement infrarouge comportant un capteur d'infrarouges (22), servant notamment à mesurer la température de la membrane du tympan, ou un réflectomètre auriculaire comportant une source acoustique (40) et un micro (42), servant notamment à mesurer l'impédance de la membrane du tympan.

14. Appareil selon les revendications 13 et 8, **caractérisé en ce que** le capteur d'infrarouges (22) est disposé à l'extrémité du conducteur de rayonnement/lumière (20).

15. Appareil selon la revendication 14, **caractérisé en ce que** le détecteur (30) et le capteur d'infrarouges (22) présentent un boîtier commun.

16. Appareil selon la revendication 14 ou 15, **caractérisé en ce que** le détecteur (30) et le capteur d'infrarouges (22) sont intégrés sur la même puce.

17. Appareil selon les revendications 13 et 1, **caractérisé en ce que** le détecteur (30) et le micro (42) présentent un boîtier commun.

18. Appareil selon les revendications 13 et 1 ou selon la revendication 17, **caractérisé en ce que** le détecteur (30) et le micro (42) sont intégrés sur la même puce.
